# EUROPÄISCHE PATENTSCHRIFT

(11) **EP 3 017 748 B1**
(45) Veröffentlichungstag und Bekanntmachung des Hinweises auf die Patenterteilung: **20.11.2019**
(21) Anmeldenummer: 15188263.6
(22) Anmeldetag: 02.10.2015
(51) Int. Cl.: A61B 1/00, A61B 1/04

(54) **ADAPTER FÜR DIE ENDOSKOPIE**
ADAPTER FOR ENDOSCOPY
ADAPTATEUR POUR L'ENDOSCOPIE

(30) Priorität: 10.11.2014 DE 102014222880
(43) Veröffentlichungstag der Anmeldung: 11.05.2016
(73) Patentinhaber: XION GmbH, 13127 Berlin (DE)
(72) Erfinder: Müller, Holger, 16548 Glienicke-Nordbahn (DE); Laser, Helmut, 13156 Berlin (DE)
(74) Vertreter: Eisenführ Speiser

(56) Entgegenhaltungen:
- US-A- 5 498 230
- US-A- 6 030 339
- US-A- 6 080 101

## Beschreibung

### Gegenstand der Erfindung

Die Erfindung betrifft einen Adapter zur Kopplung zwischen wechselbaren Endoskopen und Kameraköpfen. Solche Adapter werden aufgrund dieser Funktion auch Zwischenadapter, Koppler, Endoskopkoppler oder Kamerakoppler genannt.

### Hintergrund der Erfindung

Bei der Endoskopie müssen Geräte, welche mit dem Patienten oder dem sterilen medizinischen Personal in Verbindung kommen, zur Vermeidung von Infektionen steril gehalten werden. Deswegen werden vor der Anwendung Endoskope sterilisiert und Kameraköpfe und Kamerakabel mit einem nicht wiederverwendbaren sterilen Überzug aus Kunststoff versehen. Zur sicheren Trennung zwischen dem sterile Äußeren und dem nicht sterilen Inneren solcher Überzüge können sterile Adaptersysteme eingesetzt werden. Dadurch können an den Kamerakopf angeschlossene sterile Endoskope schnell und bequem ausgetauscht werden ohne die sterile Barriere zum nicht sterilen Kamerakopf unterbrechen zu müssen.

Aus US 5433221A ist ein steriler Überzug mit optischem Fenster bekannt.

Die Verbindung eines Kamerakopfes mit einem sterilen Endoskop durch einen speziellen sterilen Zwischenadapter und einen sterilen Überzug des Kamerakopfes ist aus US 5498230 und US 5792045 bekannt. Aus US 4756304A ist ein spezieller steriler Zwischenadapter mit Durchführungen für Beleuchtungslicht bekannt.

Ein Kameraadapter für Stereoendoskope ist aus US 5702350 bekannt.

Aus EP 2563203 A4 ist ein Adapter für zweikanalige Stereoendoskope bekannt.

Aus DE 9300529 U1 ist ein spezieller steriler Zwischenadapter für Stereoendoskope bekannt.

Aus US 6030339 A ist ein Zwischenadapter gemäß der Präambel von Anspruch 1 bekannt.

### Zusammenfassung der Erfindung

Ziel dieser Erfindung ist es einen verbesserten Adapter und ein verbessertes Adaptersystem, umfassend ein Endoskop, einen Adapter und einen Kamerakopf bereitzustellen.

Erfindungsgemäß wird diese Aufgabe gelöst von einem Adapter zur lösbaren und wiederarretierbaren Kopplung eines Endoskops mit einem Kamerakopf gemäß Anspruch 1.

Durch den Aufbau des Adapters können zahlreiche Verbesserungen erzielt werden. Ein Aspekt der Erfindung ist es, dass der optische und mechanische Aufbau verkürzt werden kann. Dadurch werden die optische Qualität der Abbildung und die praktische Handhabung des Endoskops verbessert. Ein weiterer Aspekt ist, dass die Festigkeit, Steifigkeit und Präzision der Kopplung zwischen Endoskop und Kamerakopf erhöht werden kann. Ein weiterer Aspekt der Erfindung ist, dass eine geringere Baugröße ermöglicht werden kann. Hierdurch wird das System aus Endoskop, Adapter und Kamerakopf handlicher. Weiterhin kann der Adapter besser sterilisierbar und einfacher handhabbar sein, so dass auch unter Zeitdruck keine Fehlbedienungen des Adapters auftreten. So kann in einer Ausgestaltung des Adapters beispielsweise ein Wechsel eines sterilen Überzugs vereinfacht sein. Der Adapter ist sowohl für zweidimensionale (2D-) und dreidimensionale (3D-) Endoskopie geeignet. In der dreidimensionalen (3D-) Endoskopie entstehen durch die Verwendung von Stereoendoskopen besondere Anforderungen an die Kopplung zwischen Kamera und Endoskop. In Abhängigkeit von der Bauart des Stereoendoskops können eine hohe Kopplungsgenauigkeit und eine Sicherung gegen Verdrehen erforderlich sein. In einer Ausgestaltung des Adapters ist der Adapter für die Verwendung in Stereoendoskopen geeignet und weist hierfür insbesondere eine Verdrehsicherung auf, die es ermöglicht ein steriles System mit Stereoendoskop, Adapter und Kamerakopf herzustellen, da die Verdrehsicherung sicherstellt, dass die optischen Kanäle des Stereoendoskops zu den optischen Kanälen des Kamerakopfes ausgerichtet sind. Hierdurch kann ein Justierungsaufwand reduziert werden. Weiterhin weist eine Ausgestaltung des Adapters nur wenige mechanische Teile auf, wodurch Kosten reduziert werden können. Weiterhin ist somit ein einfacherer Aufbau des Adapters möglich und auch eine einfachere Sterilisierung.

Die Innen- und Außenwand können in wenigstens einer lateralen Ebene konzentrisch zueinander angeordnet sein. Konzentrisch ist hier als symmetrisch um eine gemeinsame Mitte angeordnet zu verstehen. Die Form der Innen- und Außenwand ist hierbei weitgehend frei und nur durch die Symmetriebedingung eingeschränkt. In einer möglichen alternativen Ausgestaltung kann die Anordnung auch unsymmetrisch sein.

In einer möglichen Ausgestaltung des Adapters ist der Adapter zwischen einem auf einer distalen Seite des Adapters anordenbaren Endoskop und einem auf einer proximalen Seite des Adapters anordenbaren Kamerakopf keimdicht, um so eine sterile Seite von einer unsterilen Seite zu trennen und die sterile Seite auf diese Weise steril zu halten. Keimdicht ist hier derart zu verstehen, dass der Adapter keine Keime von einer Seite auf die andere Seite des Adapters dringen lässt. Somit können also keine Keime von einer Seite auf die andere Seite des Adapters kommen. Hierzu kann beispielsweise ein Überzug über den Kamerakopf gezogen werden. Typischerweise ist der Kamerakopf auf der unsterilen Seite und wird mit einem Überzug eingehüllt, der auf einer Innenseite des Überzugs unsteril ist und auf der Außenseite des Überzugs steril ist. Beispielsweise kann ein Endoskop auf der sterilen distalen Seite des Adapters angeordnet und ein Kamerakopf auf der unsterilen proximalen Seite des Adapters. In diesem Fall ist der unsterile Kamerakopf von dem Überzug umgeben, der auf seiner Innenseite unsteril und auf seiner Außenseite steril ist, wodurch leicht ein anderes Endoskop am Adapter arretierbar ist, ohne die Sterilität eines Adaptersystems aus Endoskop, Adapter und Kamerakopf zu beeinträchtigen.

In einer Ausgestaltung des Adapters kann wenigstens einer der Freiräume einen entlang axialer Richtung variierenden Querschnitt aufweisen. Die Variation entlang axialer Richtung des Querschnitts kann beispielsweise einen konischer Freiraum ergeben, kann aber alternativ auch entlang axialer Richtung erst den Querschnitt vergrößern, dann wieder verkleinern und schließlich wieder vergrößern, so dass die Verengung des Freiraums beispielsweise als Befestigungsnocken verwendet werden kann. Eine Ausführung des Freiraums mit Befestigungsnocken ist besonders bevorzugt, wenn wenigstens ein Teil des Materials des Adapters ein reversibel verformbares Material, beispielsweise ein Kunststoffmaterial aufweist. Der Adapter kann aus einem Kunststoffmaterial sein, das reversibel verformbar ist oder Teile des Adapters können aus solch einem Material sein und andere Teile aus nicht verformbaren Materialien.

Der Freiraum kann beispielsweise zylinderförmig, hohlzylindermantelförmig sein oder eine andere geometrische Form haben. Unter zylinderförmig sind hier alle Formen zu verstehen, die zwei irgendwie geartete Grundflächen aufweisen, die einen axialen Abstand voneinander haben und über den axialen Abstand miteinander verbunden sind. Zylinderförmig umfasst beispielsweise kreiszylinderförmige, prismatische oder weitere zylinderförmige Formen.

Der Adapter hat ein distales und ein proximales Ende. Am distalen Ende des Adapters wird typischerweise ein Endoskop angeschlossen und am proximalen Ende ein Kamerakopf. In einer Ausgestaltung des Adapters liegt wenigstens ein distales Ende der Innen- und/oder Außenwand in einer ersten Ebene mit einem distalen Ende des Adapters. Alternativ oder zusätzlich kann auch wenigstens ein proximales Ende der Innen- und/oder Außenwand in einer zweiten Ebene mit einem proximalen Ende des Adapters liegen. Der Adapter kann auch derart ausgebildet sein, dass die beiden Enden der Innen- und Außenwand zusammen in der Ebene mit den Enden des Adapters liegen und somit die Enden des Adapters bilden. Es können auch weitere Bauteile angeordnet sein, so dass keines der Enden der Außenwand und keines der Enden der Innenwand ein Ende des Adapters bildet. Weitere Bauteile können beispielsweise Formelemente, Befestigungselemente oder Überzüge sein. Die Überzüge können beispielsweise an der Außenwand des Adapters befestigt sein oder über ein Befestigungselement an die Außenwand des Adapters gepresst werden, so dass eine keimdichte Verbindung entsteht. Formelemente können dazu dienen die Form der Innen- und/oder Außenwand fortzusetzen.

In einer möglichen Ausgestaltung des Adapters weist wenigstens einer der Freiräume wenigstens eine Koppelfläche auf. Die Koppelfläche ist dazu ausgebildet mit einer anderen Koppelfläche zu koppeln. Beispielsweise kann einer der Freiräume eine Oberflächenkontur aufweisen, die mit einer Oberflächenkontur einer Koppelfläche eines zu koppelnden Endoskops oder Kamerakopfs derart abgestimmt ist, dass die Koppelflächen des Freiraums des Adapters und des zu koppelnden Endoskops oder Kamerakopfs beim ineinanderschieben koppeln. Die Kopplung kann hierbei beispielsweise durch Halte- und/oder Reibungskräfte bewirkt sein. Alternativ kann die Koppelstelle als Spielpassung mit mechanischem Kopplungsspiel ausgelegt sein. Alternativ oder zusätzlich können die Koppelflächen auch magnetisch sein oder ein magnetisches Material aufweisen, so dass eine Kopplung über magnetische Wechselwirkung möglich ist. Jede Koppelfläche kann mehrere Teilkoppelflächen aufweisen. In einem gekoppelten Zustand können alle Teilkoppelflächen des Freiraums im Eingriff mit Teilkoppelflächen des zu koppelnden Endoskops oder Kamerakopfs stehen. Alternativ kann auch nur in einem teilgekoppelten Zustand ein Teil der Teilkoppelflächen des Adapters mit entsprechenden Teilkoppelflächen des zu koppelnden Endoskops oder Kamerakopfs im Eingriff stehen. Das heißt im teilgekoppelten Zustand können Teilkoppelflächen zu den korrespondierenden Teilkoppelflächen des Kopplungspartners, d.h. des Endoskops oder Kamerakopfs, einen Abstand aufweisen, der größer als das Kopplungsspiel des Adapters ist. Die Koppelfläche kann sich axial von einem distalen Ende des Freiraums bis zu einem proximalen Ende des Freiraums erstrecken. Die Koppelfläche kann auch nur einen Teil des Freiraums umfassen. Die axiale Ausdehnung der Koppelfläche kann somit auch nicht im Eingriff stehende Teilkoppelflächen umfassen.

Erfindungsgemäß ist der Adapter ausgebildet sicherzustellen, dass in einem Zustand, in dem der Adapter mit Endoskop und Kamerakopf gekoppelt ist, die in einem jeweiligen der Freiräume hineinragenden Teile des proximalen Endoskop-Endes und des distalen Kamerakopf-Endes entlang axialer Richtung des Adapters um mindestens 50% einer axialen Länge wenigstens eines der Freiräume überlappen. Die in den jeweiligen Freiraum hineinragenden Teile können mit der Koppelfläche oder einer oder mehreren Teilkoppelfläche des Freiraums koppeln und gekoppelte Teile bilden.

Unter gekoppelten Teilen werden alle Teile des Bereichs des proximalen Endoskop-Endes und des distalen Kamerakopf-Endes entlang axialer Richtung des Adapters verstanden, die zwischen einer äußersten proximalen und einer äußersten distalen Kopplungsstelle des Freiraums liegen. Der gekoppelte Teil kann auch nicht in Eingriff stehende bzw. nicht gekoppelte Teilflächen umfassen, beispielsweise kann ein Teilbereich der die Koppelfläche bildendenden Oberflächenkontur eines der Freiräume derart ausgeführt sein, dass dieser Teilbereich nicht zur Kopplung beiträgt.

Die Innenwand, Außenwand, Verbindungswand und/oder Kopplungswand können entlang axialer Richtung und/oder entlang des Polarwinkels variierende Wandstärken aufweisen. Hierdurch kann auch der Querschnitt eines der Freiräume oder beider Freiräume entlang axialer Richtung variieren. Beispielsweise kann in einem Fall der Querschnitt eines Freiraums konstant bleiben und der des anderen Freiraums variieren.

Die Innenwand, Außenwand, Verbindungswand und/oder Kopplungswand kann eine oder mehrere Planflächen aufweisen. Die Innenwand, Außenwand, Verbindungswand und/oder Kopplungswand kann auch eine oder mehrere Rotationsflächen, d.h. geometrische Flächen, die durch Rotation um eine senkrecht zu einer Ebene angeordnete Achse einer in der Ebene liegenden Kurve erzeugt werden, wie beispielsweise Zylinderflächen bzw. Zylindermantelflächen, Kegelflächen, Hyperboloidflächen und/oder Paraboloidflächen aufweisen. Die Innenwand, Außenwand, Verbindungswand und/oder Kopplungswand kann auch einer oder mehrere Flächen aufweisen, die nicht durch Rotation einer Kurve um eine senkrecht zu einer Ebene angeordnete Achse einer in der Ebene liegenden Kurve erzeugt werden können, wie beispielsweise Prismenflächen, Pyramidenflächen, Polyederflächen oder Freiformflächen.

Die Innenwand, Außenwand, Verbindungswand und/oder Kopplungswand können im Zustand, in dem der Adapter mit Endoskop und Kamerakopf gekoppelt ist, im Eingriff mit Teilen des Endoskops oder Kamerakopfes stehen um beispielsweise einen axialen Anschlag zu bilden. Die Innenwand, Außenwand, Verbindungswand und/oder Kopplungswand kann im Zustand, in dem der Adapter mit Endoskop und Kamerakopf gekoppelt ist, auch in einer Entfernung zu Teilen des Endoskops oder Kamerakopfes stehen und somit keinen axialen Anschlag bilden.

Weiterhin kann der Adapter derart ausgebildet sein, dass Koppelflächen von Endoskop, Kamerakopf und Adapter im Zustand, in dem der Adapter mit Endoskop und Kamerakopf gekoppelt ist, in axialer Richtung um mehr als 50% der axialen Länge der Außenwand des Adapters mit der Außenwand überlappen. Infolge des Überlapps ist der Adapter in radialer Richtung partiell doppelwandig.

Aus dem Überlapp resultiert eine geringe axiale Baulänge. Das betrifft nicht nur den Adapter selbst, sondern das Gesamtsystem aus Endoskop, Adapter und Kamerakopf. Durch den Überlapp kann somit ein sehr günstiges Verhältnis aus Führungslänge und Führungsdurchmesser an den Koppelflächen von Endoskop und Kamerakopf realisiert werden. Dadurch kann Klemmen und Verkanten der Kopplung vermieden werden. In einer Ausgestaltung ist der Adapter derart ausgebildet, dass beim Koppeln des Adapters die Koppelflächen leicht gegeneinander gleiten, wodurch ein leichtes zuverlässiges Koppeln des Adapters mit dem Endoskop und dem Kamerakopf ermöglicht wird.

Erfindungsgemäß weist die Kopplungswand wenigstens ein optisches Fenster auf, das ausgebildet ist Strahlung im optischen Wellenlängenbereich zu transmittieren. Das wenigstens eine optische Fenster kann mit einem Fenstermittelpunkt um einen Kopplungswandmittelpunkt herum angeordnet sein. Es können mehrere optische Fenster auf der Kopplungswand angeordnet sein, beispielsweise zwei optische Fenster, die auf die Strahlengänge eines Stereoendoskops abgestimmt sind. Es können auch mehrere optische Fenster so angeordnet sein oder ein optisches Fenster einen so großen Durchmesser haben, dass mehrere Strahlengänge, beispielsweise eines Stereoendoskops, durch das optische Fenster oder die optischen Fenster transmittiert werden können. Die proximale Seite und die distale Seite des Adapters sind durch das optische Fenster oder die optischen Fenster hermetisch oder wenigstens keimdicht voneinander getrennt, da das oder die optischen Fenster nur Strahlung, jedoch beispielsweise keine Keime durchlassen, so dass die proximale und die distale Seite des Adapters steril voneinander getrennt werden können. Die Kopplungswand kann in einer nicht zur Erfindung gehörenden Variante auch eine Öffnung für einen Lichtdurchlass aufweisen. In diesem Fall können durch die Öffnung Keime durchdringen. Die Öffnung kann alternativ ein optisches Fenster oder mehrere optische Fenster aufweisen, welches oder welche die proximale Seite und die distale Seite des Adapters hermetisch oder wenigstens keimdicht voneinander trennen.

In einer nicht zur Erfindung gehörenden Ausgestaltung des Adapters, ist der Adapter für die Verwendung eines Endoskops ohne optischen Bildaustritt ausgebildet. In dieser Ausgestaltung kann die Bildübertragung als Signalübertragung zum Kamerakopf oder externen Komponenten über elektrische oder optische Signalleitungen oder über Funkübertragung erfolgen. Der Adapter kann zur Signalübertragung Öffnungen, Stecker, Buchsen, Signalleitungen, Übertragungselektronik oder für elektromagnetische Strahlung transparente Fenster aufweisen.

In einer Ausgestaltung des Adapters kann der Adapter für die Verwendung zur Energieübertragung ausgebildet sein. Der Adapter kann zur Energieübertragung Heatpipes, Rohrleitungen, Glasfasern, Peltierelemente, Leitungsanschlüsse, Ferritkerne, Öffnungen, Stecker, Buchsen, elektrische Leitungen, elektrische Bauteile, elektronische Bauteile, Wellen, Getriebe, Kupplungen oder für elektromagnetische Strahlung transparente Fenster aufweisen. Eine Energieübertragung kann in jeder Form insbesondere thermisch, elektrisch, mechanisch, induktiv oder als Strahlung erfolgen. Die Bauelemente zur Energieübertragung können Teil des Adapters sein oder mit diesem lösbar und wiederarretierbar verbunden sein.

In einer möglichen Ausgestaltung ist der Adapter für eine Übertragung von wenigstens einem Medium, d.h. eines Stoffes in irgendeinem Aggregatzustand oder irgendeiner Erscheinungsform, wie beispielsweise eines Gases, eines Plasmas, einer Flüssigkeiten, eines Pulvers und/oder eines Feststoffes ausgebildet. Der Adapter kann zur Leitung von einem Medium eine oder mehrere Bauelemente, wie beispielsweise Rohrleitungen, Leitungsanschlüsse, Öffnungen, Förderschnecken, Pumpen und/oder dergleichen aufweisen. Die Bauelemente zur Medienübertragung können Teil des Adapters sein oder mit diesem lösbar und wiederarretierbar verbunden sein.

In einer Ausgestaltung kann der Adapter einen Medienspeicher aufweisen, der mit Bauelementen zur Leitung der Medien verbunden sein kann. Solche Behälter zur Speicherung der Medien können beispielsweise als Druckbehälter für Gase oder Speicherbehälter für Flüssigkeiten oder Pulver ausgebildet sein. Die Medienspeicher können Teil des Adapters sein oder mit diesem lösbar und wiederarretierbar verbunden sein.

In einer möglichen Ausgestaltung des Adapters weist der Adapter eine Energiequelle zum Betrieb von Kameraelektronik, Signalübertragung, Motoren, Sensoren, Kühlung, Beleuchtung oder dergleichen auf, welche dazu ausgebildet ist Energie durch Energieumwandlung bereitzustellen. Insbesondere können solche Energiequellen photovoltaische Elemente, Induktionsspulen, Batterien, Akkumulatoren, Turbinen, Generatoren, thermoelektrische Elemente, Druckbehälter oder dergleichen aufweisen. Die Bauelemente der Energiequelle können Teil des Adapters sein oder mit diesem lösbar und wiederarretierbar verbunden sein. In einer Ausgestaltung kann der Adapter dazu ausgebildet sein Bewegungen zu übertragen oder zu erzeugen. Der Adapter kann hierzu Bewegungsübertragungselemente, wie beispielsweise Kupplungen, Wellen, Zugstangen, Seilzüge, Getriebe, pneumatische oder hydraulische Leitungen, und/oder dergleichen aufweisen, die beispielsweise die distale mit der proximalen Seite des Adapters beispielsweise über Öffnungen im Adapter oder dergleichen verbinden. Zur Erzeugung von Bewegungen kann der Adapter Antriebe wie beispielsweise Motoren, Elektromotoren oder Druckluftantriebe aufweisen. Die Elemente zur Übertragung und Erzeugung von Bewegungen können Teil des Adapters sein oder mit diesem lösbar und wieder arretierbar verbunden sein.

In einer möglichen Ausgestaltung ist der Adapter zur Führung und/oder Befestigung von Instrumenten durch den Adapter ausgebildet. Der Adapter kann hierfür Öffnungen, Führungsrollen, Führungsrohre, Lager, Führungen, Befestigungselemente und/oder weitere zur Führung oder Befestigung von Instrumenten durch den Adapter geeignete Komponenten aufweisen.

Der Adapter kann dafür ausgebildet sein, dass austauschbare optische für elektromagnetische Strahlung transparente Fenster, Energiespeicher, Energiequellen, Energiesenken, Leitungen, Motoren, Medienspeicher, Datenspeicher, Sensoren und/oder Übertragungselemente während eines Betriebs des Adapters austauschbar sind.

In einer Ausgestaltung können ein oder mehrere optische Fenster zum Austauschen oder zum Austauschen während des Betriebs des Adapters ausgeführt sein, um Reinigung, Sterilisation, Reparatur oder Wartung zu vereinfachen.

In einer Ausgestaltung des Adapters kann der zur Kopplung mit wenigstens einem Teil eines distalen Kamerakopf-Endes ausgebildete Freiraum mindestens eine Koppelfläche aufweisen, die ausgebildet ist mit mindestens einer Koppelfläche des distalen Kamerakopf-Endes zu koppeln. Weiterhin kann der zur Kopplung mit wenigstens einem Teil eines proximalen Endoskop-Endes ausgebildete Freiraum mindestens eine Koppelfläche aufweisen, die ausgebildet ist mit mindestens einer Koppelfläche des proximalen Endoskop-Endes zu koppeln. In einer möglichen Ausgestaltung weist die Innenwand sowohl auf ihrer Innenseite, als auch auf ihrer Außenseite Koppelflächen auf, die ausgebildet sind Endoskop und Kamerakopf anzukoppeln. Die Koppelflächen können beispielsweise zylinderförmig sein.

In einer möglichen Ausgestaltung des Adapters ist mindestens eine der Koppelflächen zylinderförmig ausgebildet. Zylinderförmig ist hier im weitesten Sinne zu verstehen, d.h. als die abgeleitete Zylindermantelfläche von einem Zylinder, der von zwei parallelen, ebenen, kongruenten Flächen und einer Mantelfläche bzw. Zylinderfläche definiert wird. Insbesondere umfasst der Begriff zylinderförmig also auch prismatische, aber auch kreiszylindrische Flächen. In einem Adaptersystem, weisen Endoskop, Kamerakopf und Adapter vorzugsweise jeweils Koppelflächen auf, die bevorzugt aufeinander abgestimmt sind, so dass eine Kopplung zwischen den Koppelflächen eine Kopplungsstärke zwischen Adapter und Endoskop bzw. Kamerakopf erhöht. Hierdurch kann eine stärkere Kopplung zwischen Endoskop, Adapter und Kamerakopf hergestellt werden, die insgesamt ein stabileres System ermöglicht.

In einer möglichen Ausgestaltung des Adapters ist mindestens eine der Koppelflächen an mindestens zwei Stellen koppelnd ausgeführt, d.h. die Koppelfläche hat zwei Teilkoppelflächen, die mit einem axialen Abstand zueinander angeordnet sind. In einer alternativen Ausgestaltung können auch zwei oder mehrere Teilkoppelflächen aneinandergrenzen oder einen axialen und/oder radialen Abstand zueinander aufweisen. Die Teilkoppelflächen können zylinderförmige Teilkoppelflächen sein. Die Teilkoppelflächen können auch konisch ausgebildet sein. Es können auch mehrere aneinandergrenzende Teilkoppelfächen einen Winkel zueinander aufweisen, so dass sich beispielsweise für den von den Teilkoppelflächen umschlossenen Freiraum eine Querschnittsverengung oder Querschnittsverbreiterung entlang der axialen Richtung ergibt. Die Teilkoppelflächen können auch Freiformflächen aufweisen. Die Teilkoppelflächen können derart ausgebildet sein, dass sie einen in axialer Richtung variierenden Querschnitt eines Freiraums erzeugen, wodurch ein Einführen eines zu koppelnden Endoskops oder Kamerakopfs in den Freiraum vereinfacht werden kann. In diesem Fall sind Hinterschneidungen zu vermeiden.

In einer Ausgestaltung des Adapters kann mindestens eine der Koppelflächen oder deren Teilkoppelflächen derart ausgebildet sein, dass sich ein entlang axialer Richtung variierender Querschnitt in wenigstens einem der Freiräume ergibt.

In einer möglichen Ausgestaltung des Adapters ist mindestens eine der Koppelflächen konisch ausgebildet. In einer weiteren Ausgestaltung ist wenigstens eine Teilkoppelfläche einer Koppelfläche konisch ausgebildet.

In einer Ausgestaltung können die Koppelflächen zylinderförmige Flächen mit verschiedenen Durchmessern sein, welche durch konische Übergangsflächen miteinander verbunden sind. Hierbei liegt beim Koppeln des Adapters anfangs größeres mechanisches Spiel vor, welches sich später beim weiteren Koppeln verringert wenn alle zylinderförmigen Koppelflächen in Eingriff kommen. Diese Anordnung lässt sich mit sehr geringer Kraft schließen und ist sicher gegen Verkanten und Verklemmen.

In einer möglichen Ausgestaltung weist der Adapter wenigstens eine Verdrehsicherung auf, die ausgebildet ist ein Verdrehen eines mit dem Adapter gekoppelten Endoskops und/oder Kamerakopfs zu verhindern. Die Verdrehsicherung kann beispielsweise ein Loch, eine Bohrung, oder dergleichen in Verbindung mit einem auf dieses oder diese angepassten Bolzen, Stift, oder dergleichen sein.

In einer Ausgestaltung des Adapters kann eine Verdrehsicherung aktivierbar und deaktivierbar sein. Ein Umschalten zwischen einem aktiviertem und einem nicht aktiviertem Zustand kann beispielsweise realisiert werden indem die Verdrehsicherung bewegliche Bauelemente aufweist, die bei geschlossener Kopplung aus dem Eingriffsbereich der Verdrehsicherung heraus bewegt werden können. In einer Ausgestaltung kann das bewegliche Bauelement so ausgeführt sein, dass die Verdrehsicherung eine Rastung bildet.

In einer möglichen Ausgestaltung des Adaptersystems sind an den Adapter verdrehsicher ausgeführte Endoskope verdrehsicher und nicht verdrehgesicherte ausgeführte Endoskope nicht verdrehgesichert anschließbar. Eine solche unterschiedliche Ausführung kann beispielsweise erfolgen indem verdrehgesicherte Endoskope ein verdrehsicherndes Element wie eine Passbohrung oder eine radiale Nut aufweisen, welche in einen Stift, Bolzen oder dergleichen am Adapter greift und ein nicht verdrehgesichertes Endoskop an Stelle des verdrehsichernden Elementes einen Freiraum aufweist, welcher ein gegenseitiges Verdrehen von Adapter und Endoskop ermöglicht.

In einer Ausgestaltung des Adaptersystems können an den Adapter verdrehsicher ausgeführte Kameraköpfe verdrehsicher und nicht verdrehgesicherte ausgeführte Kameraköpfe nicht verdrehgesichert anschließbar sein.

In einer möglichen Ausgestaltung des Adapters weist der Adapter Koppelflächen mit Teilkoppelflächen auf, die keine Rotationsflächen sind, d.h. geometrische Flächen, die nicht durch Rotation um eine senkrecht zu einer Ebene angeordnete Achse einer in der Ebene liegenden Kurve erzeugt werden können. Bei dieser Auslegung kann durch die gebrochene Rotationssymmetrie eine Verdrehsicherung des Adapters zu einem zu koppelndem Endoskop und/oder zu einem zu koppelndem Kamerakopf erzielt werden. Die Verdrehsicherung für ein Adaptersystem aus Adapter und Endoskop oder Kamerakopf kann sich also auch aus der Form des Freiraums und der Form des Endoskops oder Kamerakopfes ergeben, indem diese eine Form haben, die keine Rotationsfläche bildet, d.h. indem eine Drehung in einem eingeschobenen Zustand des Endoskops oder Kamerakopfes in einen der Freiräume des Adapters aufgrund der aufeinander abgestimmten Formen verhindert wird.

In einer Ausgestaltung des Adapters kann der Adapter wenigstens eine Arretierungsvorrichtung aufweisen, die ausgebildet ist das Endoskop und/oder den Kamerakopf lösbar und wiederarretierbar am Adapter zu arretieren.

In einer möglichen Ausgestaltung weist der Adapter einen sterilen Überzug zur Abdeckung des Kamerakopfes auf. Der Überzug ist ausgebildet, den Kamerakopf steril zu umschließen. Der Überzug kann am Adapter, beispielsweise an der Außenwand befestigt sein. Alternativ kann die Außenwand auch eine Befestigungsvorrichtung aufweisen, an der ein Überzug befestigt werden kann.

In einer Ausgestaltung kann der Adapter mindestens eine optische Leitung zur Übertragung von Beleuchtungslicht zwischen proximalem Ende des Adapters und distalem Ende des Adapters aufweisen. Im Adaptersystem, das vorzugsweise das Endoskop, den Adapter und den Kamerakopf aufweist, kann das Beleuchtungslicht durch den Adapter zum distalen Ende des Endoskops übermittelt werden und dort auf ein zu beleuchtendes Objekt gestrahlt werden, welches Licht reflektiert, das durch das Endoskop und den Adapter zum Kamerakopf geführt wird.

In einer möglichen Ausgestaltung weist der Adapter mindestens eine elektrische Leitung zur Übertragung von elektrischen Signalen oder elektrischer Energie beispielsweise zwischen proximalem Ende des Adapters und distalem Ende des Adapters auf. Die Energie kann beispielsweise zur Messung, zum Betreiben von Lichtquellen oder zur Elektrostimulation verwendet werden.

Der Adapter kann auch eine oder mehrere optische und elektrische Leitungen aufweisen.

Das Adaptersystem kann ausgebildet sein elektromagnetische Strahlung zu übertragen und/oder zu erzeugen. Zur Erzeugung elektromagnetischer Strahlung kann der Adapter, das Endoskop oder der Kamerakopf eine Strahlungsquelle aufweisen. Die Strahlungsquelle kann Leuchtdioden, Laserdioden, Laser, Infrarotstrahler, Radiosender oder dergleichen aufweisen. Zur Übertragung elektromagnetischer Strahlung kann der Adapter Öffnungen, Fenster, Lichtleiter, Hohlleiter, Antennen oder dergleichen aufweisen. Die Elemente zur Erzeugung und Übertragung elektromagnetischer Strahlung können fest oder lösbar und wiederarretierbar mit dem Adapter, dem Endoskop oder Kamerakopf verbunden sein. Die elektromagnetische Strahlung kann beispielsweise zur diagnostischen oder therapeutischen Bestrahlung oder Beleuchtung, zur Bilderzeugung, sowie für Messungen wie Abstandsmessungen, Größenmessung, Topografiemessungen, Radarmessungen und/oder Radarkartierung dienen.

In einer möglichen Ausgestaltung ist das Adaptersystem zur Messung mindestens einer physikalischen Größe ausgebildet. Hierzu kann der Adapter, das Endoskop oder der Kamerakopf einen Sensor aufweisen. Solche gemessenen physikalische Größen können beispielsweise Temperatur, Druck, Zeit, Abstände, Längen, Bewegungen, Lage, Orientierung, Horizontlage, Feuchte, Beleuchtungsstärke oder Schalldruck sein. Geeignete Sensoren können beispielsweise Hall-Sensoren, Thermofühler, Mikrophone, GPS-Empfänger, Photometer, Triangulationssensoren sein. Der Sensor kann fest oder lösbar und wiederarretierbar mit dem Adapter, dem Endoskop oder Kamerakopf verbunden sein. Die Messung können beispielsweise der Erfassung von Benutzungsdauern, Sterilisationsdaten oder Verschleißdaten dienen, welche automatisch oder manuell auswertbar seien können.

In einer Ausgestaltung kann das Adaptersystem einen Datenspeicher zur Speicherung von Messdaten, Nutzungsdaten, Verschleißdaten, Behandlungsdaten, Bildinformationen, ausführbaren Programmen oder dergleichen aufweisen. Der Datenspeicher kann fest oder lösbar und wiederarretierbar mit dem Adapter, dem Endoskop oder Kamerakopf verbunden sein.

Die in einem Datenspeicher gespeicherten Daten können durch geeignete Bauelemente erzeugt, ausgelesen, weiterverarbeitet, übertragen, überschrieben, gelöscht oder als Programm ausgeführt werden. Genannte Bauelemente können einen Bestandteil des Adapters, Endoskops oder Kamerakopfes bilden oder keinen Bestandteil des Adaptersystems bilden.

Auf dem Datenspeicher gespeicherte Programme können beispielsweise zur Programmierung einer Verarbeitungseinheit durch die den Datenspeicher aufweisende Komponente des Adaptersystems dienen. Dadurch kann eine Verarbeitungseinheit für verschiedenste Systemkonfigurationen und/oder Betriebszuständen des Adaptersystems passend programmiert werden. Genannte Verarbeitungseinheit kann einen Bestandteil des Adapters, Endoskops oder Kamerakopfes bilden oder keinen Bestandteil des Adaptersystems bilden.

In einer möglichen Ausgestaltung weist das Adaptersystem eine Verarbeitungseinheit auf, die zur Ausführung von Programmen ausgebildet ist. Solche ausführbaren Programme können beispielsweise der Signalübertragung, Signalverarbeitung, Steuerung von Ladevorgängen, Bildverarbeitung, Bilderfassung, Telekommunikation, Bewegungssteuerung, Steuerung, Messung oder Datenerfassung für diagnostische oder therapeutische Anwendungen dienen. Die Verarbeitungseinheit kann beispielsweise Mikroprozessoren, digitale Signalprozessoren (DSPs), FPGAs oder ASICs aufweisen. Die Verarbeitungseinheit kann fest oder lösbar und wiederarretierbar mit dem Adapter, dem Endoskop oder Kamerakopf verbunden sein.

In einer Ausgestaltung kann das Adaptersystem wenigstens ein Element zur Erkennung der Position durch ein Navigationssystem aufweisen. Solche Elemente zur Erkennung der Position können Magnete, Gyrosensoren, Transponder, Reflektoren, Markierungen oder optisch sichtbare Formelemente sein. Elemente zur Erkennung der Position können fest oder lösbar und wiederarretierbar mit dem Adapter, dem Endoskop oder Kamerakopf verbunden sein.

In einer möglichen Ausgestaltung weist das Adaptersystem ein Bedienelement zur Steuerung und/oder Aktivierung von Kamerafunktionen oder Endoskopfunktionen auf. Bedienelemente können beispielsweise ausgebildet sein einen Fokus einzustellen, Funktionen in einer Menüstruktur auszuwählen und zu steuern ein Standbild oder Video aufzuzeichnen, wiederzugeben, oder einen Weißabgleich vorzunehmen. Bedienelemente können fest oder lösbar und wiederarretierbar mit dem Adapter, dem Endoskop oder Kamerakopf verbunden sein.

In einer Ausgestaltung kann das Adaptersystem zur Verwendung mit einem Manipulator oder Robotersystem ausgebildet sein. Hierzu kann das Adaptersystem Halterungen und Schnittstellen für Signalübertragung, Steuerungen, Medienübertragung, Kraftübertragung, Positionserfassung, Betriebszustanderkennung und dergleichen aufweisen. Halterungen und Schnittstellen können fest oder lösbar und wiederarretierbar mit dem Adapter, dem Endoskop oder Kamerakopf verbunden sein.

Weiterhin betrifft die Erfindung auch die Verwendung eines Adapters zur Kopplung eines Endoskops oder Stereoendoskops mit einem Kamerakopf. Das Stereoendoskop und der Kamerakopf sind aufgrund der doppelten optischen Leitung zueinander auszurichten. Hierfür wird bevorzugt die Ausgestaltung mit der Verdrehsicherung verwendet. Die Verdrehsicherung kann Teil des Adapters sein oder Teil eines Adaptersystems aus Endoskop, Adapter und Kamerakopf, wobei an Endoskop, Adapter und Kamerakopf Teile der Verdrehsicherung angeordnet sind.

Die Erfindung betrifft auch ein Adaptersystem aus Endoskop, Adapter und Kamerakopf. Das proximale Ende des Endoskops, der Adapter und das distale Ende des Kamerakopfs sind so aufeinander abgestimmt, dass der Adapter mit wenigstens einem Teil des proximalen Endes des Endoskops und mit einem Teil des distalen Endes des Kamerakopfs koppeln kann. Hierdurch wird der Strahlengang durch das Adaptersystem nur geringfügig verlängert, da nur ein relativ dünnes optisches Fenster den Strahlengang verlängert und ansonsten das Endoskop und der Kamerakopf mit dem Adapter so gekoppelt sind, dass der Adapter das Endoskop und den Kamerakopf trennt. Die Trennung kann keimdicht sein und einen Überzug für den Kamerakopf und an diesen angeschlossene Komponenten aufweisen, um die proximale und die distale Seite steril voneinander zu trennen und so ein nach außen steriles Adaptersystem zu schaffen.

In einer möglichen Ausgestaltung des Adaptersystems weist der Kamerakopf, der Adapter und/oder das Endoskop eine Energiesenke zur Wärmeabfuhr auf. Insbesondere können solche Energiesenken Kühlkörper, Verdampfer, Strahler, thermoelektrische Elemente oder Wärmeübertrager aufweisen. Die Bauelemente der Energiesenke können Teil des Adapters, des Endoskops und/oder des Kamerakopfes sein oder lösbar und wiederarretierbar mit einem oder mehreren von diesen verbunden sein.

In einer Ausgestaltung kann das Adaptersystem ein Element zur Heizung aufweisen. Ein Element zur Heizung kann beispielsweise ein Beschlagen optischer Komponenten verhindern. Ein Element zur Heizung kann Bestandteil von Endoskop, Adapter oder Kamerakopf sein oder mit Endoskop, Adapter oder Kamerakopf lösbar und wiederarretierbar verbunden sein.

In einer möglichen Ausgestaltung des Adaptersystems weist der Kamerakopf, der Adapter und/oder das Endoskop einen Energiespeicher auf. Insbesondere können solche Energiespeicher Brennstoffzellen, Akkumulatoren, Wärmespeicher, Federn oder Schwungräder aufweisen. Die Bauelemente des Energiespeichers können Teil des Adapters, des Endoskops und/oder des Kamerakopfs sein oder lösbar und wiederarretierbar mit einem oder mehreren von diesen verbunden sein.

In einer Ausgestaltung des Adaptersystems kann der Adapter, das Endoskop und/oder der Kamerakopf eine Lichtquelle aufweisen. Die Lichtquelle kann Teil des Adapters, des Endoskops und/oder des Kamerakopfs sein oder mit einem oder mehreren von diesen lösbar und wiederarretierbar verbunden sein.

In einer möglichen Ausgestaltung des Adaptersystems weist der Kamerakopf, der Adapter und/oder das Endoskop eine Energiequelle zum Betreiben von Kameraelektronik, Signalübertragung, Motoren, Sensoren, Kühlung, Beleuchtung oder dergleichen auf, welche ausgebildet ist Energie durch Energieumwandlung bereitzustellen. Insbesondere können solche Energiequellen photovoltaische Elemente, Induktionsspulen, Batterien, Akkumulatoren, Turbinen, Generatoren, thermoelektrische Elemente oder dergleichen aufweisen. Die Bauelemente der Energiequelle können Teil des Adapters, des Endoskops und/oder des Kamerakopfs sein oder mit einem oder mehreren von diesen lösbar und wiederarretierbar verbunden sein.

Besonders vorteilhaft ist die Verwendung eines Adapters zur Kopplung eines Stereoendoskops mit einem Kamerakopf, da bei Stereoendoskopen eine Verlängerung der Adaptersystemlänge mit großem technischen Aufwand und einer deutlichen Verschlechterung der Bildqualität verbunden ist. Deswegen muss bei bekannten Stereoendoskopen nach dem Stand der Technik entweder ohne sterilen Adapter gearbeitet werden oder aus Platzgründen kann kein Bedienring zur Fokussierung an der üblichen und günstigsten Position nahe dem distalen Ende des Kamerakopfes angebracht werden. Mit anderen Worten, konnte bisher der bewährte und weit verbreitete Grundaufbau eines zweidimensionalen (2D-) Endoskopiesystems mit einer manuellen Fokussierung nahe dem distalen Ende nur dann für die Stereoendoskopie verwendet werden, wenn auf einen sterilen Zwischenadapter verzichtet wurde. Die Erfindung hingegen ermöglicht den Aufbau eines solchen sterilen stereoendoskopischen Systems. Dadurch kann mit der Erfindung ein wesentlich höherer Bedienkomfort bei sterilen stereoskopischen Arbeiten erzielt werden als bisher üblich.

Bei zweikanaligen Stereoendoskopen ist neben der axialen Ausrichtung auch eine horizontale Ausrichtung von Kamerakopf und Stereoendoskop erforderlich. Der Adapter weist hierfür in einer möglichen Ausgestaltung eine Verdrehsicherung auf, die ein gegenseitiges Verdrehen von Endoskop, Adapter und Kamerakopf verhindern. Beispiele einer Verdrehsicherung sind Stifte, die bei gekoppeltem Adapter in Löchern, Langlöchern oder Nuten positioniert sind. Die Verdrehsicherung kann auch Vorsprünge, die in Vertiefungen greifen aufweisen. Weiterhin können Schrauben, Federklemmungen, Klemmungen, Rastverbindungen, Magnetverbindungen und dergleichen so ausgeführt sein, dass sie eine Verdrehsicherung bilden. Die Verdrehsicherung kann auch durch einen Bajonettverschluss erzeugt werden. Weiterhin kann eine Verdrehsicherung erzielt werden, wenn Koppelflächen oder Teilkoppelflächen als nicht-Rotationsflächen ausgebildet sind.

Neben den Koppelflächen weist die Erfindung eine Arretierungsvorrichtung mit Arretierelementen zur lösbaren und wiederarretierbaren Arretierung des Endoskops mit dem Adapter und weitere zur lösbaren und wiederarretierbaren Arretierung des Kamerakopfes mit dem Adapter auf. Hierzu können alle bekannten Arretierelemente wie Schrauben, Federklemmungen, Klemmungen, Bajonettverschlüsse, Rastverbindungen, Magnetverbindungen und dergleichen verwendet werden. In einer Ausgestaltung des Adaptersystems aus Endoskop, Adapter und Kamerakopf weist das Endoskop nahe seines proximalen Endes eine Schraube mit Feststellmutter zur Arretierung am Adapter auf, während der Kamerakopf nahe seines distalen Endes eine gefederte Bajonettverbindung zur Arretierung des Kamerakopfes am Adapter aufweist. Bedienelemente für die Arretierung des Endoskops können alternativ am Adapter angebracht werden. Außerdem können Bedienelemente für die Arretierung des Kamerakopfes alternativ am Adapter oder Kamerakopf angebracht werden.

Bevorzugt ist der Adapter so ausgebildet, dass beim Koppeln des Adapters mit dem Endoskop oder dem Kamerakopf zuerst die Koppelflächen soweit in Eingriff kommen, dass beide zu koppelnde Bauteile axial ausgerichtet sind und radiale Verschiebungen verhindert werden, während axiale Verschiebung und Rotation noch möglich sind. Die Verdrehsicherung oder Verdrehsicherungen kommt oder kommen, sofern vorhanden, bevorzugt erst danach in Eingriff und der Kopplungsvorgang wird durch Betätigen der Arretierungsvorrichtung oder Arretierungsvorrichtungen zum Arretieren des Adapters an dem Endoskop oder an dem Kamerakopf abgeschlossen. Der Adapter ermöglicht große Führungslängen an den Koppelflächen und die zu koppelnden Bauteile können bereits zu Beginn des Koppelns des Adapters gut geführt werden. Auch wenn das Endoskop oder der Kamerakopf in wahllosem Rotationswinkel verdreht mit dem Adapter verbunden werden, kann nach teilweisen Koppeln des Adapters die richtige Orientierung in der Rotationsrichtung durch gegenseitiges Verdrehen leicht und ohne hohe Konzentration des Bedieners hergestellt werden. Die Verdrehsicherung kann derart ausgebildet sein, dass bei einem wahllosen Verdrehen der mit leichtem Druck zusammengeführten zu koppelnden Bauteile der Adapter in der richtigen Drehstellung blockiert oder rastet. Dadurch kann insbesondere bei Stereoendoskopen die Handhabung des Adapters wesentlich leichter und intuitiver erfolgen und das Risiko von Fehlbedienungen deutlich verringert werden.

Weiterhin kann durch Verwendung des Adapters in einem Adaptersystem aus Endoskop, Adapter und Kamerakopf auch die radiale Baugröße verringert werden. Da sich bei geeigneter Ausführung die Koppelflächen am Endoskop, Kamerakopf und Adapter gegenseitig mechanisch unterstützen, können im Bereich der Koppelflächen geringe Materialstärken eingesetzt werden und trotzdem eine hohe Festigkeit und Steifigkeit des Adapters erreicht werden. Der Adapter kann zur Einmalverwendung ausgebildet sein, beispielsweise aus einem Kunststoff. In diesem Fall ist der Festigkeitsgewinn besonders vorteilhaft. Die Erhöhung der Steifigkeit kann durch ein präziseres Koppeln gleichzeitig die Bildqualität verbessern.

Weiterhin kann durch die mittels des Adapters erzielte geringe Baugröße des Adaptersystems aus Endoskop, Adapter und Kamerakopf ein sehr günstiges Verhältnis der Hebellängen von Adapter zu denen des Adaptersystems erzielt werden. Dadurch kann ein weiterer Gewinn an Festigkeit und Steifigkeit gegeben sein.

Weiterhin ermöglicht der Adapter einen sehr einfachen mechanischen Aufbau mit geringer Anzahl an beweglichen Bauteilen, wodurch hygienische Anforderungen wie Sterilität und Sauberkeit besser erfüllbar sind. Darüber hinaus erhöht sich dadurch die Produktzuverlässigkeit und es verringern sich Fertigungskosten.

Der Adapter kann steril zur Einmalverwendung oder sterilisierbar zur Mehrfachverwendung ausgeführt werden. Der Adapter kann lösbar oder permanent mit einem sterilen Überzug für Kamerakopf und Kabel verbunden sein. Eine solche lös- und wiederarretierbare Verbindung kann durch einen elastischen Ring oder Klebeband am Überzug realisiert werden. Weiterhin kann eine solche lösbare Verbindung als Rastverbindung, Bajonettverschluss, Schraubverschluss oder Magnetbefestigung ausgeführt werden. Derartige sterile Überzuge können zur einmaligen oder mehrmaligen Verwendung ausgelegt werden.

Es können auch Durchführungen für lichtleitende Fasern und elektrische Kontakte in den Adapter integriert werden um Beleuchtungslicht und elektrische Signale über den Adapter zu Übertragen.

Der Adapter kann Sensoren zur Identifizierung von angeschlossen Endoskopen oder dem Adapter beziehungsweise die Kombination aus beidem durch den Kamerakopf aufweisen. Der Adapter kann auch Leitungen zur Durchleitung von Signalen für derartige Identifizierungen aufweisen. So können beispielsweise Fenster zum Durchlass elektromagnetischer Signale am oder im Adapter angeordnet werden. Der Adapter kann auch Transponder zum Senden und/oder Empfangen elektromagnetischer Signale aufweisen. Der Adapter kann auch elektronische Schaltkreise aufweisen. Der Adapter kann ausgebildet sein ein gekoppeltes Endoskop und/oder einen gekoppelten Kameraköpfe zu identifizieren. Der Adapter kann ausgebildet sein solche Identifizierungen auszuwerten, zu verarbeiten, umzuwandeln und/oder weiter zu senden. Der Adapter kann ausgebildet sein eine eigene Identifizierung an das gekoppelte Endoskop und/oder den gekoppelten Kamerakopf zu senden. Die Identifizierung des Adapters kann von Endoskop und/oder Kamerakopf zum Anpassen von für ein Adaptersystem spezifischen Parametern verwendet werden.

Neben der Verwendung als steriler oder sterilisierbarer Adapter kann der Adapter auch für nicht sterile Anwendungen verwendet werden. Eine derartige nicht sterile Verwendung ist beispielsweise die Verwendung zur Adaption verschiedener Adaptersysteme oder zur Erzielung besserer Wartungsmöglichkeiten durch Austauschbarkeit von Komponenten.

Die Erfindung soll nun anhand von in den Figuren schematisch abgebildeten Ausführungsbeispielen näher erläutert werden.

### Kurze Beschreibung der Zeichnungen

Figur 1 zeigt eine schematische Schnittdarstellung eines ersten Ausführungsbeispiels eines Adapters mit gekoppeltem Kamerakopf und Endoskop;
Figur 2 zeigt ein zweites Ausführungsbeispiels eines Adapters mit gekoppeltem Kamerakopf und Endoskop;
Figur 3 zeigt einen vergrößerten Ausschnitt aus Figur 2;
Figur 4 zeigt eine Schnittdarstellung des zweiten Ausführungsbeispiels eines Adapters;
Figur 5 zeigt eine perspektivische Darstellung mit Blickrichtung auf das proximale Ende des zweiten Ausführungsbeispiels des Adapters;
Figur 6 zeigt eine perspektivische Darstellung mit Blickrichtung auf das distale Ende des zweiten Ausführungsbeispiels des Adapters;
Figur 7 zeigt eine perspektivische Darstellung mit Blickrichtung auf das proximale Ende eines Endoskops;
Figur 8 zeigt eine perspektivische Darstellung eines Kamerakopfs;
Figur 9 zeigt eine schematische Darstellung eines Ausführungsbeispiels eines Bajonettverschlusses am Kamerakopf;
Figur 10 zeigt eine schematische Schnittdarstellung durch eine an zwei Stellen koppelnde Koppelfläche;
Figur 11 zeigt eine schematische Schnittdarstellung eines dritten Ausführungsbeispiels eines Adapters mit gekoppeltem Kamerakopf und Endoskop;
Figur 12 zeigt eine perspektivische Darstellung mit Blickrichtung auf das proximale Ende eines Endoskops mit einer eine Verdrehsicherung erzeugenden Koppelfläche.

### Detaillierte Beschreibung

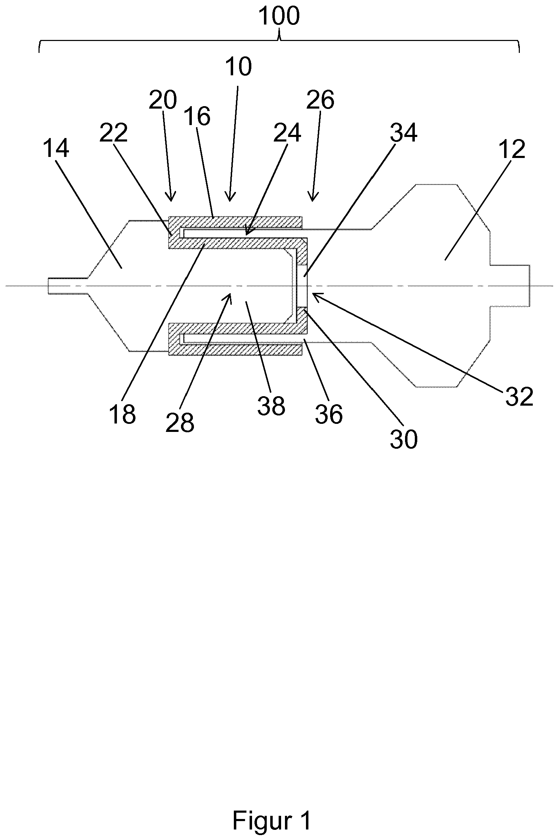

Figur 1 zeigt eine schematische Schnittdarstellung eines ersten Ausführungsbeispiels des Adapters 10 mit angekoppeltem Kamerakopf 12 und Endoskop 14, d.h. eines Adaptersystems 100. Der Kamerakopf 12 ist über den Adapter 10 lös- und wiederarretierbar mechanisch und optisch mit dem Endoskop 14 verbunden.

Der Adapter 10 hat eine Außenwand 16 und eine Innenwand 18, die im ersten Ausführungsbeispiel des Adapters 10 konzentrisch zueinander angeordnet sind und an ihrem distalen Ende 20 über eine Verbindungswand 22 miteinander verbunden sind. Hierdurch entsteht in diesem Ausführungsbeispiel zwischen Außenwand 16 und Innenwand 18 ein hohlzylindermantelförmiger auf der proximalen Seite des Adapters offener Freiraum 24. Alternativ kann auch eine andere Form eines Freiraums 24 gebildet werden, beispielsweise mit variierendem Querschnitt entlang der axialen Richtung, so dass entlang der axialen Richtung verschiedene laterale Abstände zwischen Außenwand 16 und Innenwand 18 entstehen. Im dargestellten Beispiel ist das distale Ende 20 der Außenwand 16 und Innenwand 18 auch das distale Ende des Adapters 10. Alternativ kann beispielsweise auch eine der Wände 16 oder 18 auf der distalen Seite länger sein, so dass die Wände 16 und 18 kein gemeinsames distales Ende haben (nicht gezeigt). Es kann auch beispielsweise die Verbindungswand 22 nicht am distalen Ende 20, sondern in unmittelbarer Umgebung des distalen Endes 20 angeordnet sein, so dass sich ein kleiner Überstand einer oder der beiden Wände 16 und 18 bildet.

Die Innenwand 18 erstreckt sich zwischen ihrem distalen Ende 20 und einem proximalen Ende 26, welches im dargestellten Beispiel das proximale Ende des Adapters 10 ist. Am proximalen Ende 26 der Innenwand 18 wird ein von der Innenwand 18 umschlossener Freiraum 28 durch eine Kopplungswand 30 verschlossen, so dass der von der Innenwand 18 umschlossene Freiraum 28 auf der distalen Seite des Adapters 10 offen ist. Die Kopplungswand 30 hat im dargestellten Beispiel eine Öffnung 32, in der ein optisches Fenster 34 angeordnet ist. Das optische Fenster 34 dient dazu Strahlung im sichtbaren Wellenlängenbereich zwischen der distalen und proximalen Seite des Adapters 10 zu übertragen.

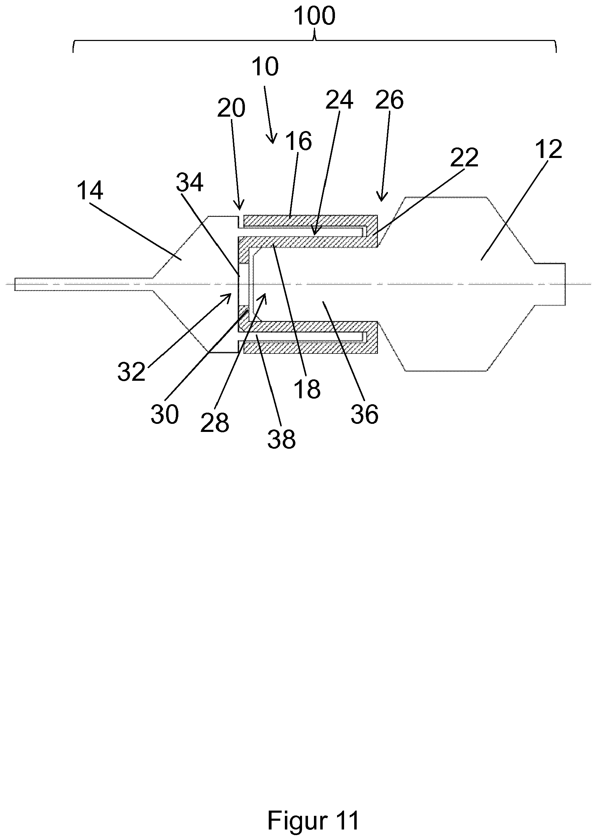

Im dargestellten Beispiel ist der von Außenwand 16 und Innenwand 18 gebildete Freiraum 24 derart ausgebildet, dass ein Teil eines distalen Kamerakopf-Endes 36 in diesen hineinragen und gekoppelt werden kann, d.h. der Freiraum 24 ist auf die Form eines Teils des distalen Kamerakopf-Endes 36 abgestimmt. Der von der Innenwand 18 umschlossene Freiraum 28 ist in diesem Fall derart ausgebildet, dass ein Teil eines proximalen Endoskop-Endes 38 in diesen hineinragen und gekoppelt werden kann, d.h. der Freiraum 28 ist auf die Form eines Teils des proximalen Endoskop-Endes 38 abgestimmt. Alternativ kann auch Freiraum 24 zur Kopplung des proximalen Endoskop-Endes 38 ausgebildet und hierfür auf der distalen Seite des Adapters 10 offen sein (s. Fig. 11) und Freiraum 28 zur Kopplung des distalen Endoskop-Endes 36 ausgebildet und hierfür auf der proximalen Seite des Adapters 10 offen sein (s. Fig. 11).

Mit dem Aufbau des Adapters 10 in Fig. 1 kann eine hermetische oder zumindest keimdichte Trennung zwischen proximaler und distaler Seite des Adapters 10 erzielt werden, wodurch es möglich ist eine sterile von einer unsterilen Seite zu trennen und so die sterile Seite steril zu halten.

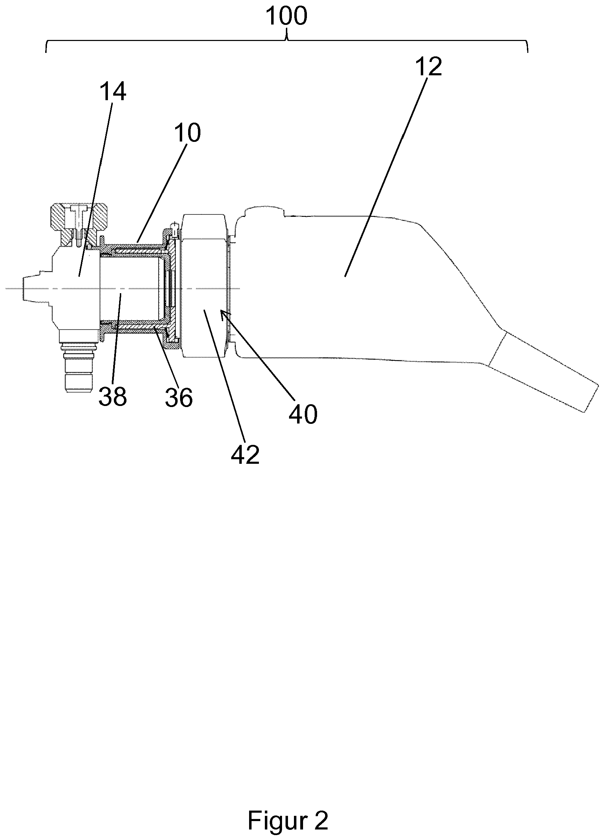

Figur 2 zeigt ein zweites Ausführungsbeispiels eines Adaptersystems 100 mit Adapter 10 in einem mit Kamerakopf 12 und Endoskop 14 gekoppelten Zustand. Das Adaptersystem 100 umfasst eine Fokussierungsvorrichtung 40, die es über einen von einem Nutzer bedienbaren Fokussierungsbedienring 42 ermöglicht den Strahlengang innerhalb des Adapters 10 auf eine im Kamerakopf 12 enthaltene Bildaufnahmevorrichtung (nicht gezeigt) zu fokussieren. Durch den kompakten Aufbau des Adaptersystems 100 kann der Fokussierungsbedienring 42 der Fokussierungsvorrichtung 40 an einer ergonomisch und technisch günstigsten Stelle nahe dem distalen Kamerakopfendes 36 angebracht werden.

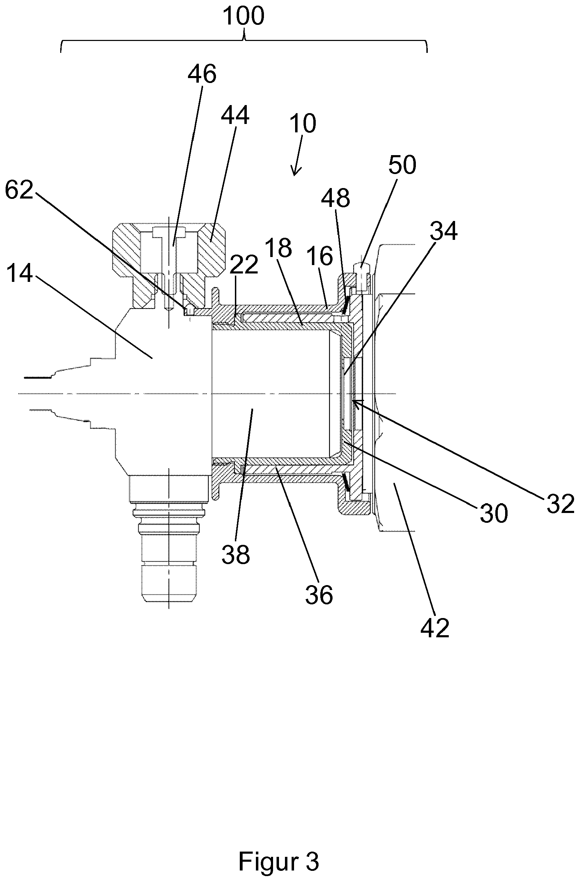

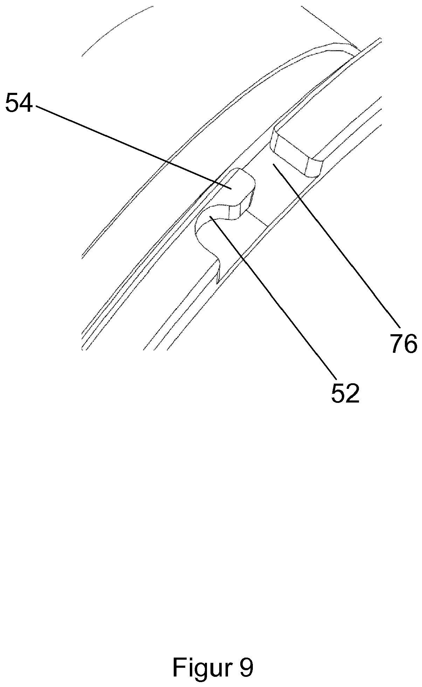

Figur 3 zeigt einen vergrößerten Ausschnitt aus Figur 2. Das Endoskop 14 hat eine Feststellmutter 44 zur Arretierung des Adapters 10 am Endoskop 14. Um eine gute Umspülung während der Sterilisation zu erreichen, kann die Feststellmutter 44 vor der Sterilisation gelöst werden. Eine Verlustsicherung 46 gegen Verlieren der Feststellmutter 44 hält die Feststellmutter 44 im gelösten Zustand mit dem Endoskop 14 zusammen. Eine am Kamerakopf 12 angebrachte Tellerfeder 48 erzeugt eine mechanische Drucckraft zwischen Adapter 10 und dem Kamerakopf 12. Dadurch rastet der proximale Bolzen 50 zur Feststellung des Kamerakopfes 12 mit der Kraft der Tellerfeder 48 in der in Figur 9 gezeigten Aufnahmefläche 52 des Bajonettverschlusses 54 ein.

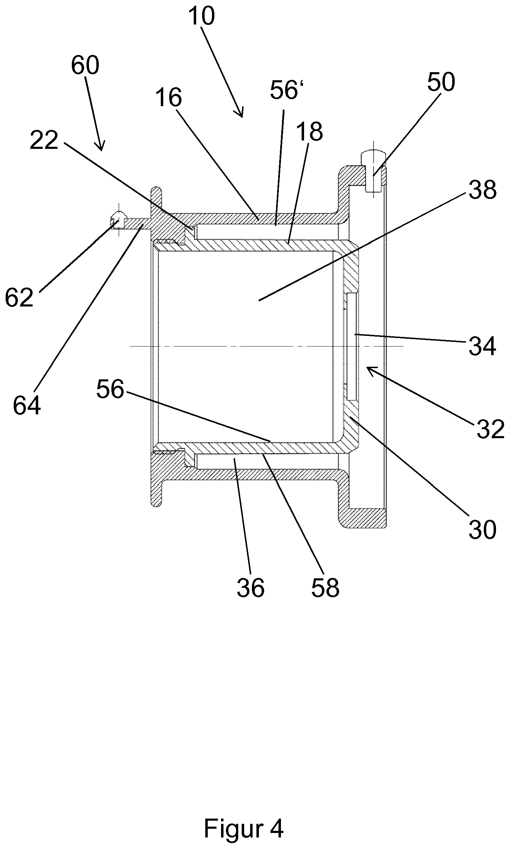

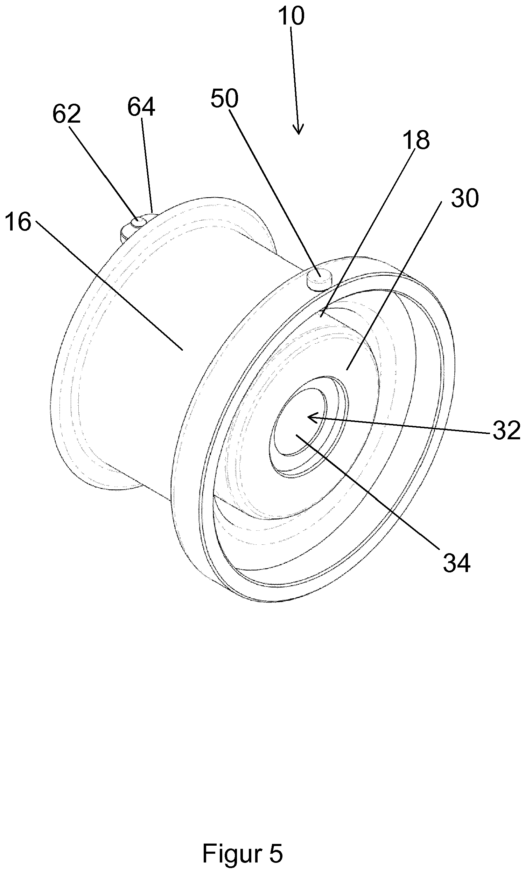

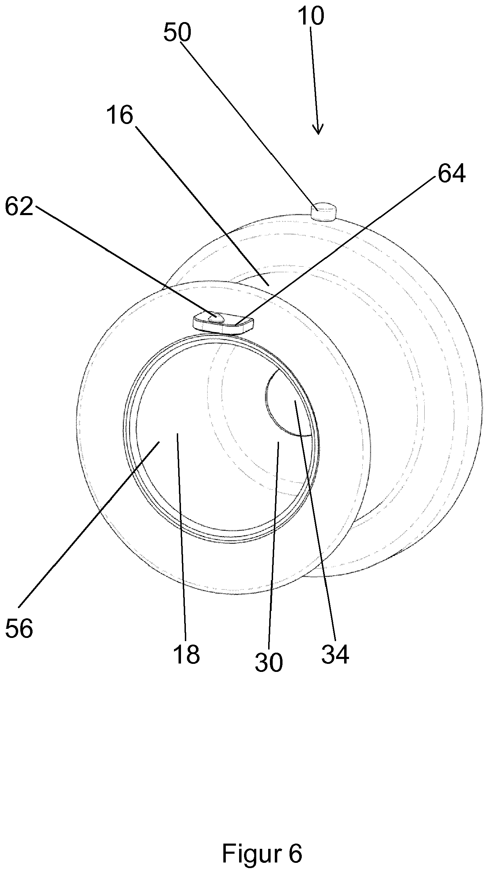

Figur 4 zeigt eine Schnittdarstellung des zweiten Ausführungsbeispiels eines Adapters 10. In Figur 5 und Figur 6 sind perspektivische Darstellungen dieses zweiten Ausführungsbeispiels gezeigt. Die Außenwand 16 ist im dargestellten Beispiel durch die Verbindungswand 22 lösbar mit der Innenwand 18 verbunden. Nahe dem proximalen Ende des Adapters 10 wird der von der Innenwand 18 umschlossene Freiraum 38 durch die Kopplungswand 30 verschlossen. In der Öffnung 32 für den Lichtdurchlass ist das optische Fenster 34 angeordnet. Die Innenwand 18 hat im dargestellten Beispiel eine innere Koppelfläche 56 und eine äußere Koppelfläche 58. Die innere Koppelfläche 56 ist ausgebildet mit einem Teil des proximalen Endoskop-Endes 38 zu koppeln und die äußere Koppelfläche 58 ist ausgebildet mit einem Teil des distalen Kamerakopf-Endes 36 zu koppeln. Auch die Außenwand 16 weist auf ihrer Innenseite eine innere Koppelfläche 56' auf, die ausgebildet ist mit einem Teil des distalen Kamerakopf-Endes 36 gekoppelt zu werden. Alternativ kann die Innenseite der Außenwand 16 auch ohne Koppelfläche ausgeführt sein. Es können auch mehrere Teilkoppelflächen auf den Innenseiten der Innenwand 18 und Außenwand 16 und auf der Außenseite der Innenwand 18 angeordnet sein. Die Teilkoppelflächen können axial und/oder radial bzw. ggf. lateral voneinander getrennt angeordnet sein, so dass nur an bestimmten Flächen oder Stellen eine Kopplung zwischen Endoskop 14 und Adapter 10 und Kamerakopf 12 und Adapter 10 entsteht. Ein lateraler Abstand der Teilkoppelflächen kann bei einer von einer kreiszylindrischen Form abweichenden zylinderförmigen Form, wie beispielsweise einer prismatischen Form, auftreten.

Nahe dem distalen Ende 60 des Adapters 10 weist der Adapter 10 einen distalen Bolzen 62 zur Feststellung des Endoskops 14 am Adapter 10 auf. Der distale Bolzen 62 ist im dargestellten Beispiel an einer Verdrehsicherungslasche 64 angebracht, welche ein Verdrehen des an den Adapter 10 angeschlossenen Endoskops 14 verhindert. Nahe dem proximalen Ende des Adapters 10 befindet sich der proximale Bolzen 50 zur Arretierung des Kamerakopfes 12 am Adapter 10. Der Adapter 10 kann wie dargestellt aus zwei Drehteilen gefertigt und dadurch zerlegbar sein. Es ist jedoch auch eine Fertigung aus nur einem oder mehr als zwei Teilen möglich. Die mehreren Teile können auch Drehteile sein.

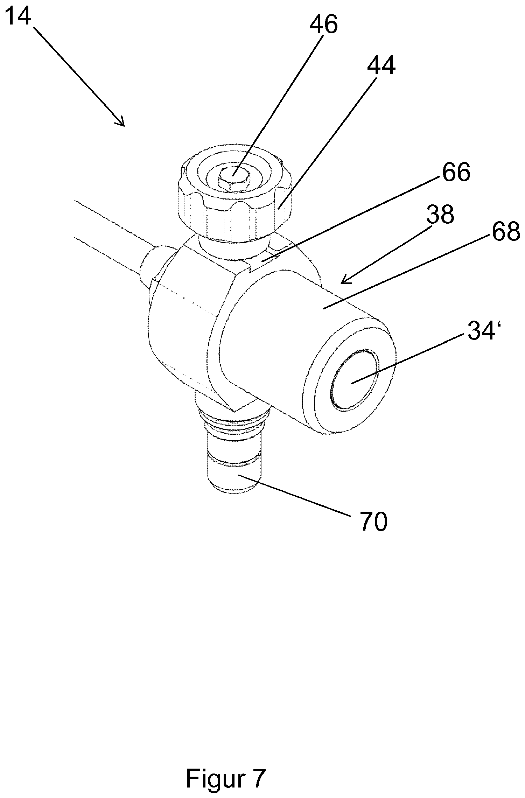

Figur 7 zeigt eine perspektivische Darstellung mit Blickrichtung auf das proximale Ende eines Endoskops 14. Das Endoskop 14 weist die Feststellmutter 44 zur Arretierung des Endoskops 14 am Adapter 10 auf. Die Verlustsicherung 46 verhindert, dass die sich die Feststellmutter 44 in einem gelösten Zustand vom Endoskop 14 trennt und verloren geht. Im gelösten Zustand sind die Gewindeflanken der Feststellmutter 44 einem Sterilisationsmittel, wie beispielsweise Desinfektionsmittel oder einem anderen Desinfektionsmedium, zugänglich. Die Form einer Nut 66 ist auf die Verdrehsicherungslasche 60 (s. Fig. 6) abgestimmt, wodurch bei gekoppeltem Adapter 10 ein gegenseitiges Verdrehen von Endoskop 14 und Adapter 10 verhindert wird. Die Koppelfläche 68 des Endoskops 14 ist in diesem Ausführungsbeispiel kreiszylinderförmig ausgebildet, kann aber auch andere Formen wie beispielsweise eine zylinderförmige Form, eine konische Form oder andere Formen haben. Zylinderförmig ist hier im weitesten Sinne zu verstehen und umfasst beispielsweise auch prismatische Formen. Die Form kann auch in axiale Richtung Querschnittsänderungen aufweisen, wobei die Form des proximalen Endoskop-Endes 38 auf die Form des Freiraums 28 des ersten Ausführungsbeispiels des Adapters 10 (s. Fig. 1) abgestimmt ist, um eine Kopplung zu ermöglichen. Die Form des proximalen Endoskop-Endes 38 kann auch anders ausgebildet sein, um auf ein alternatives Ausführungsbeispiel des Adapters 10 abgestimmt zu sein (s. Fig. 11).

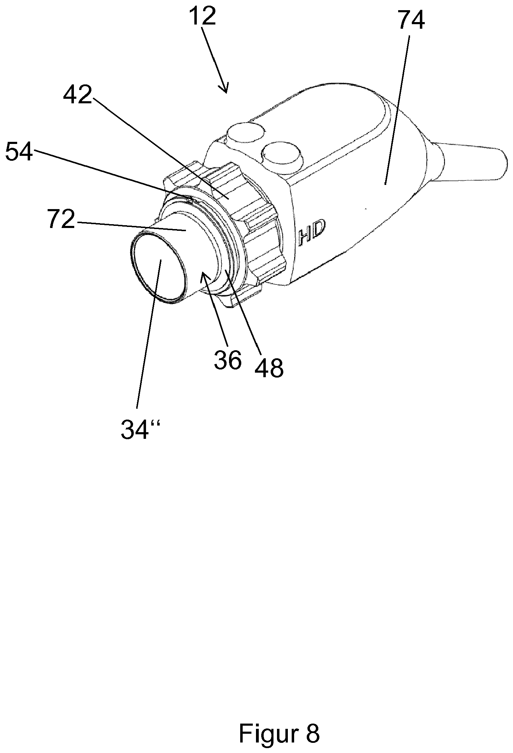

Ein optisches Fenster 34' des Endoskops 14 grenzt im mit dem Adapter 10 gekoppelten Zustand an das optische Fenster 34 des Adapters 10. Hierdurch wird der Strahlengang durch den Adapter 10 nur um die Dicke des optischen Fensters 34 des Adapters 10 verlängert. Es ist auch möglich einen Adapter 10 ohne optisches Fenster 34 auszuführen, so dass in diesem Fall das optische Fenster 34' des Endoskops 14 unmittelbar durch die Öffnung 32 des Adapters 10 an ein optisches Fenster 34" des Kamerakopfes 12 (s. Fig. 8) angrenzen kann, so dass der Adapter 10 die Länge des Strahlengangs nicht beeinflusst.

Das dargestellte Endoskop 14 hat einen Anschluss 70 für ein Lichtleiterkabel, um dem Endoskop 14 und somit im gekoppelten Zustand des Endoskop 14 mit dem Adapter 10, dem Adaptersystem 100 Beleuchtungslicht zuzuführen.

Figur 8 zeigt eine perspektivische Darstellung eines Kamerakopfes 12. Die Koppelfläche 72 des proximalen Kamerakopf-Endes 36 ist auf den zur Kopplung des proximalen Kamerakopf-Endes 36 ausgebildeten Freiraum 24 des ersten Ausführungsbeispiels des Adapters 10 (s. Fig. 1) abgestimmt und kann somit mit dem Adapter 10 gekoppelt werden. In dem Bajonettverschluss 54 kann der Adapter 10 mittels des proximalen Bolzens 50 (s. Fig. 6) arretiert werden. Durch Tellerfeder 48 wird eine Kraft für ein definiertes Einrasten des proximalen Bolzens 50 (s. Fig. 6) in den Bajonettverschluss 54 erzeugt. Der Kamerakopf 12 eignet sich durch seinen verdrehsicheren Aufbau für die Verwendung mit Stereoendoskopen (nicht gezeigt). Der Kamerakopf 12 weist nahe dem distalen Ende des Kameragehäuses 74 einen Fokussierungsbedienring 42 auf, der dazu dient einen Strahlengang auf eine im Kamerakopf 12 enthaltene Bildaufnahmevorrichtung (nicht gezeigt) zu fokussieren.

Figur 9 zeigt eine schematische Darstellung eines Ausführungsbeispiels eines Bajonettverschlusses 54 am Kamerakopf 12. Durch eine Einführungsnut 76 kann ein am Adapter 10 befindliches Befestigungselement wie beispielsweise ein Bolzen, ein Stift oder dergleichen in den Bajonettverschluss 54 eingeführt werden. Nach gegenseitigem Verdrehen von Kamerakopf 12 und Adapter 10 rastet dieses Befestigungselement in der Aufnahmefläche 52 ein. Die Rastkraft kann durch ein geeignetes federndes Element erzeugt werden.

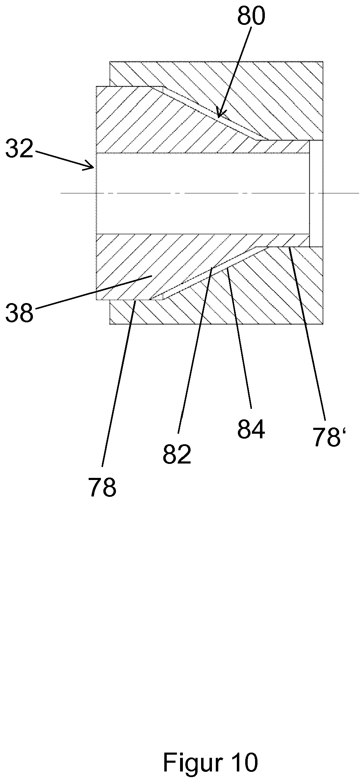

Figur 10 zeigt eine schematische Schnittdarstellung eines Teilausschnitts eines Adapters 10 durch eine an zwei Stellen koppelnde Koppelfläche 78, 78'. In einem nicht gezeigten alternativen Ausführungsbeispiel kann die Koppelfläche auch an drei oder mehr Stellen koppelnd ausgeführt sein. Im dargestellten Beispiel ist ein proximales Endoskop-Ende 38 in einen auf distaler Seite offenen Freiraum des Adapters 10 teilweise eingeschoben. Anstatt des proximalen Endoskop-Endes 38 kann alternativ auch ein distales Kamerakopf-Ende 36 in einen auf der proximalen Seite offenen Freiraum des Adapters 10 eingeschoben sein (nicht gezeigt). Das Endoskop hat eine Öffnung 32, durch die ein Strahlengang (nicht gezeigt) geführt werden kann.

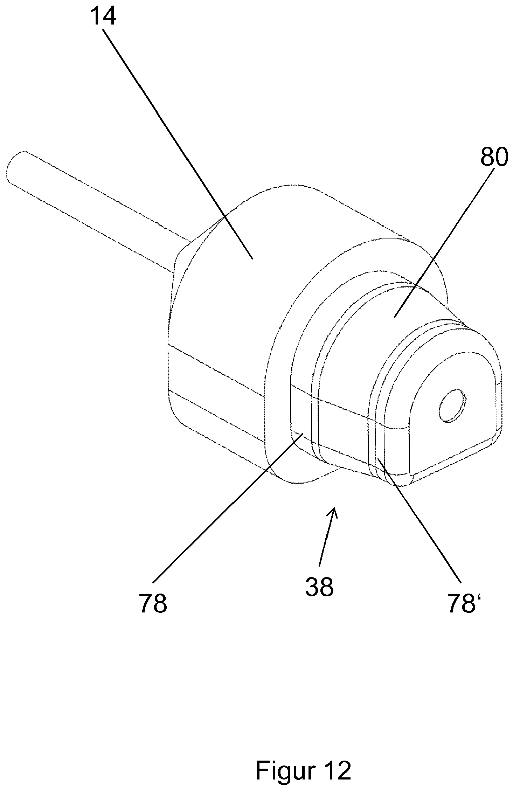

Zwei zylinderförmige Teilkoppelflächen 78 und 78' eines proximalen Endoskop-Endes 38 werden durch einen konischen Übergang 80 miteinander verbunden (s. Fig. 12). Im dargestellten Beispiel sind die Teilfläche 82 des Endoskops 14 und die Teilfläche 84 des Adapters 10 am konischen Übergang 80 nicht koppelnd ausgeführt, so dass sich zwischen den zwei koppelnden Teilkoppelflächen 78 und 78' ein nicht koppelnder axialer Abschnitt ergibt.

Es ist besonders vorteilhaft die Koppelflächen von Adapter 10, Endoskop 12 und Kamerakopf 14 in der in Fig. 10 gezeigten Weise an zwei Stellen koppelnd auszulegen. Dadurch wird eine leichte Betätigung bei geringem mechanischem Spiel ermöglicht.

Die Koppelflächen können beispielsweise magnetisch sein und durch eine magnetische Anziehung gegenüberliegender Koppelflächen koppeln. Alternativ können Koppelflächen auch mechanisch koppeln, beispielsweise aufgrund der Oberflächenkontur und zwischen den Koppelflächen auftretenden Reibungskräften. Es ist auch möglich verschiedene Koppelflächenarten, d.h. mechanisch und magnetisch koppelnde Koppelflächen zu mischen, beispielsweise kann eine an einer Stelle mechanisch und an einer anderen Stelle magnetisch koppelnde Koppelfläche verwendet werden.

Figur 11 zeigt ein drittes Ausführungsbeispiel eines Adapters 10. Im Gegensatz zu dem ersten Ausführungsbeispiel des Adapters 10 aus Fig. 1 ist der von Außenwand 16 und Innenwand 18 umschlossene hohlzylindermantelförmige Freiraum 24 im dritten Ausführungsbeispiel des Adapters 10 auf der distalen Seite offen und der von der Innenwand umschlossene Freiraum 28 auf der proximalen Seite offen. Dies wird ermöglicht, indem der Adapter 10 umgedreht wird und die auf die Freiräume 24 und 28 des Adapters 10 abgestimmten Formen des proximalen Endoskop-Endes 38 und des distalen Kamerakopf-Endes 36 auf den gedrehten Adapter 10 angepasst werden.

In den auf der distalen Seite offenen Freiraum 24ist ein Teil des proximalen Endoskop-Endes 38 eingeführt. In den auf der proximalen Seite offenen Freiraum 28 ist ein Teil des distalen Kamerakopf-Endes 36 eingeführt. Die Verbindungswand 22, die Kopplungswand 30 und das optische Fenster 34 bewirken eine sterile Trennung zwischen der distalen und proximalen Seite des Adapters 10. Der Adapter 10 ist in diesem Fall keimdicht und verhindert, dass Keime von der distalen auf die proximale Seite des Adapters oder umgekehrt gelangen. Das dritte Ausführungsbeispiel des Adapters 10 ist insbesondere für die Verwendung zum Herstellen einer stabilen Verbindung zu Kameraköpfen mit elektromotorischer Fokussierung vorgesehen.

Figur 12 zeigt eine perspektivische Darstellung mit Blickrichtung auf das proximale Ende eines Endoskops 14 bei welchem die Koppelfläche 78, 78' des Endoskops an zwei Stellen koppelnd mit zylinderförmigen Teilkoppelflächen 78, 78' ausgeführt ist, die miteinander über einen nichtkoppelnden konischen Übergang 80 verbunden sind. Die Form der Koppelfläche 78, 78' ermöglicht ein verdrehsicheres und leicht steckbares Koppeln des Endoskops 14 an einen dazu passend geformten Adapter 10 (s. Fig. 10).

### Bezuqszeichenliste

- 10: Adapter
- 12: Kamerakopf
- 14: Endoskop
- 16: Außenwand
- 18: Innenwand
- 20: distales Ende der Außenwand und Innenwand
- 22: Verbindungswand
- 24: zwischen Innen- und Außenwand liegender Freiraum
- 26: proximales Ende der Innenwand
- 28: von der Innenwand umschlossener Freiraum
- 30: Kopplungswand
- 32: Öffnung
- 34, 34', 34": optisches Fenster
- 36: distales Kamerakopf-Ende
- 38: proximales Endoskop-Ende
- 40: Fokussierungsvorrichtung
- 42: Fokussierungsbedienring
- 44: Feststellmutter
- 46: Verlustsicherung
- 48: Tellerfeder
- 50: proximaler Bolzen
- 52: Aufnahmefläche
- 54: Bajonettverschluss
- 56, 56': innere Koppelfläche
- 58: äußere Koppelfläche
- 60: distales Ende des Adapters
- 62: distaler Bolzen
- 64: Verdrehsicherungslasche
- 66: Nut
- 68: Koppelfläche des Endoskops
- 70: Anschluss für ein Lichtleiterkabel
- 72: Koppelfläche des Kamerakopfes
- 74: Kameragehäuse
- 76: Einführungsnut
- 78, 78': zweistellige Koppelfläche des Endoskops
- 80: konischer Übergang
- 82: Teilfläche des Endoskops am konischen Übergang
- 84: Teilfläche des Adapters am konischen Übergang
- 100: Adaptersystem

## Patentansprüche

1. Adapter (10) zur lösbaren und wiederarretierbaren Kopplung eines Endoskops (14) mit einem Kamerakopf (12), umfassend:
- eine sich entlang einer axialen Richtung erstreckende Außenwand (16) und eine mindestens teilweise von der Außenwand umgebene, sich entlang derselben axialen Richtung erstreckende Innenwand (18), die über eine sich zwischen der Innen- (18) und Außenwand (16) an oder in der Nähe von deren ersten axialen Enden (20) erstreckende Verbindungswand (22) miteinander verbunden sind und mit wenigstens einem lateralen Abstand voneinander angeordnet sind, so dass sich zwischen Innen- (18) und Außenwand (16) ein an einer den ersten axialen Enden (20) gegenüberliegenden zweiten axialen Seite offener Freiraum (24) ergibt und
- eine Kopplungswand (30), die einen von der Innenwand (18) umschlossenen Freiraum (28) an oder nahe eines zweiten axialen Endes (26) der Innenwand (18) verschließt, so dass der von der Innenwand (18) umschlossene Freiraum (28) auf einer dem zweiten axialen Ende (26) der Innenwand (18) gegenüberliegenden ersten axialen Seite offen ist,
wobei einer der Freiräume (24, 28) zur Kopplung mit wenigstens einem Teil eines distalen Kamerakopf-Endes (36) ausgebildet ist, und
wobei der andere der Freiräume (24, 28) zur Kopplung mit wenigstens einem Teil eines proximalen Endoskop-Endes (38) ausgebildet ist, **dadurch gekennzeichnet, dass**
der Adapter weiterhin ausgebildet ist sicherzustellen, dass sich in einem Zustand, in dem der Adapter (10) mit Endoskop (14) und Kamerakopf (12) gekoppelt ist, die in einem jeweiligen der Freiräume (24, 28) hineinragenden Teile des proximalen Endoskop-Endes (38) und des distalen Kamerakopf-Endes (36) entlang axialer Richtung des Adapters (10) um mindestens 50% einer axialen Länge wenigstens eines der Freiräume (24, 28) überlappen,
wobei die Kopplungswand (30) wenigstens ein optisches Fenster zum optischen Bildaustritt aufweist, das ausgebildet ist, Strahlung im optischen Wellenlängenbereich zu transmittieren.

2. Adapter (10) gemäß Anspruch 1, der zwischen proximale Seite des Adapters (10) und distaler Seite des Adapters (10) keimdicht ist, um so eine sterile Seite von einer unsterilen Seite zu trennen und die sterile Seite auf diese Weise steril zu halten.

3. Adapter (10) gemäß Anspruch 1 oder 2, wobei wenigstens einer der Freiräume (24, 28) einen entlang der axialen Richtung und/oder entlang des Polarwinkels variierenden Querschnitt aufweist.

4. Adapter (10) gemäß wenigstens einem der Ansprüche 1 bis 3, wobei der zur Kopplung mit wenigstens einem Teil eines distalen Kamerakopf-Endes (36) ausgebildete Freiraum (24, 28) mindestens eine Koppelfläche (56, 56', 58) aufweist, die ausgebildet ist mit mindestens einer Koppelfläche (72) des distalen Kamerakopf-Endes (36) zu koppeln und
wobei der zur Kopplung mit wenigstens einem Teil eines proximalen Endoskop-Endes (38) ausgebildete Freiraum (24, 28) mindestens eine Koppelfläche (56, 56', 58)) aufweist, die ausgebildet ist mit mindestens einer Koppelfläche (68) des proximalen Endoskop-Endes (38) zu koppeln.

5. Adapter (10) gemäß Anspruch 4, wobei mindestens eine der Koppelflächen (56, 56', 58) zylinderförmig oder hohlzylindermantelförmig ausgebildet ist.

6. Adapter (10) nach wenigstens einem der Ansprüche 4 oder 5, wobei mindestens eine der Koppelflächen (56, 56', 58) derart ausgebildet ist, dass sich ein entlang der axialen Richtung variierender Querschnitt in wenigstens einem der Freiräume (24, 28) ergibt.

7. Adapter (10) nach wenigstens einem der Ansprüche 1 bis 6, der einen sterilen Überzug zur Abdeckung des Kamerakopfes (12) aufweist, wobei der Überzug ausgebildet ist, den Kamerakopf (12) steril zu umschließen.

8. Adapter (10) nach wenigstens einem der Ansprüche 1 bis 7, wobei die Innenwand (18) sowohl auf ihrer Innenseite, als auch auf ihrer Außenseite Koppelflächen (56, 58) aufweist, die ausgebildet sind Endoskop (14) und Kamerakopf (12) anzukoppeln.

9. Adapter (10) nach wenigstens einem der Ansprüche 1 bis 8, der mindestens eine optische Leitung zur Übertragung von Beleuchtungslicht und/oder zur optischen Signalübertragung
und/oder mindestens eine elektrische Leitung zur Übertragung von elektrischen Signalen und/oder elektrischer Energie
zwischen proximaler Seite des Adapters (10) und distaler Seite des Adapters (10) aufweist.

10. Verwendung eines Adapters (10) nach wenigstens einem der Ansprüche 1 bis 9 zur Kopplung eines Stereoendoskops (14) mit einem Kamerakopf (12).

## Claims

1. An adapter (10) for the detachable and relockable coupling of an endoscope (14) with a camera head (12), comprising:
- an external wall (16), extending along an axial direction, and an internal wall (18), at least partially surrounded by the external wall and extending along the same axial direction, which are connected to one another via a connecting wall (22) extending between the internal (18) and external wall (16) at or in the vicinity of their first axial ends (20) and which are arranged at at least a lateral distance from one another, so that between internal (18) and external wall (16) there arises a free space (24) open at a second axial side opposite the first axial ends (20) and
- a coupling wall (30) which at or close to a second axial end (26) of the internal wall (18) closes a free space (28), enclosed by the internal wall (18), so that the free space (28) enclosed by the internal wall (18) is open at a first axial side opposite the second axial end (26) of the internal wall (18),
wherein one of the free spaces (24, 28) is for coupling with at least one part of a distal camera-head end (36)
and
wherein the other one of the free spaces (24, 28) is for coupling with at least one part of a proximal endoscope end (38),
**characterized in that**
the adapter furthermore is designed to ensure that, in a state in which the adapter (10) is coupled with endoscope (14) and camera head (12), the parts, of the proximal endoscope end (38) and the distal camera-head end (36), projecting in a respective free space (24, 28) overlap along an axial direction of the adapter (10) by at least 50% of an axial length of at least one of the free spaces (24, 28),
wherein the coupling wall (30) has at least one optical window for optical image emergence, which window is designed to transmit radiation in the optical wave length range.

2. An adapter (10) according to claim 1, which is germ-tight between proximal side of the adapter (10) and distal side of the adapter (10) in order thus to separate a sterile side from an unsterile side and in this manner to keep the sterile side sterile.

3. An adapter (10) according to claim 1 or 2, wherein at least one of the free spaces (24, 28) has a cross-section varying along the axial direction and/or along the polar angle.

4. An adapter (10) according to at least one of claims 1 to 3,
wherein the free space (24, 28) for coupling with at least one part of a distal camera-head end (36) has at least one coupling surface (56, 56', 58) for coupling with at least one coupling surface (72) of the distal camera-head end (36) and
wherein the free space (24, 28) for coupling with at least one part of a proximal endoscope end (38) has at least one coupling surface (56, 56', 58) for coupling with at least one coupling surface (68) of the proximal endoscope end (38).

5. An adapter (10) according to claim 4,
wherein at least one of the coupling surfaces (56, 56', 58) is in the shape of a cylinder or a casing of a hollow cylinder.

6. An adapter (10) according to at least one of claims 4 and 5,
wherein at least one of the coupling surfaces (56, 56', 58) is such that in at least one of the free spaces (24, 28) there arises a cross-section varying along the axial direction.

7. An adapter (10) according to at least one of claims 1 to 6, which has a sterile cover for covering the camera head (12), wherein the covering is designed to enclose the camera head (12) in a sterile manner.

8. An adapter (10) according to at least one of claims 1 to 7,
wherein the internal wall (18) has coupling surfaces (56, 58) on both its internal side and its external side, which are for attaching endoscope (14) and camera head (12).

9. An adapter (10) according to at least one of claims 1 to 8,
which has at least one optical line for transmitting illumination light and/or for optical signal transmission between the proximal side of the adapter (10) and the distal side of the adapter (10)
and/or at least one electrical line for transmitting electrical signals and/or electrical energy between the proximal side of the adapter (10) and the distal side of the adapter (10).

10. Use of an adapter (10) according to at least one of claims 1 to 9 for coupling of a stereo endoscope (14) with a camera head (12).

## Revendications

1. Adaptateur (10) de couplage détachable et reblocable d'un endoscope (14) à une tête (12) de caméra, comprenant :
- une paroi (16) extérieure s'étendant suivant une direction axiale et une paroi (18) intérieure, entourée au moins en partie de la paroi extérieure et s'étendant suivant la même direction axiale,
qui sont reliées l'une à l'autre par une paroi (22) de liaison s'étendant entre la paroi (18) intérieure et la paroi (16) extérieure ou à proximité de leur première extrémité (20) axiale et qui sont disposées à au moins une distance latérale l'une de l'autre, de manière à donner, entre la paroi (18) intérieure et la paroi (16) extérieure, un espace (24) libre ouvert à un deuxième côté axial opposé aux premières extrémités (20) axiales et
- une paroi (30) de couplage, qui ferme un espace (28) libre entouré de la paroi (18) intérieure à ou près d'une deuxième extrémité (26) axiale de la paroi (18) intérieure, de manière à ce que l'espace (28) libre entouré de la paroi (18) intérieure soit ouvert d'un premier côté axial opposé à la deuxième extrémité (26) axiale de la paroi (18) intérieure,
dans lequel l'un des espaces (24, 28) libres est constitué pour le couplage à au moins une partie d'une extrémité (36) distale de tête de caméra,
et
dans lequel l'autre des espaces (24, 28) libres est constitué pour le couplage à au moins une partie d'une extrémité (38) proximale d'endoscope,
**caractérisé en ce que**
l'adaptateur est constitué, en outre, pour assurer que, dans un état dans lequel l'adaptateur (10) est couplé à l'endoscope (14) et la tête (12) de caméra, les parties, pénétrant dans l'un respectif des espaces (24, 28) libres, de l'extrémité (38) proximale de l'endoscope et de l'extrémité (36) distale de la tête de la caméra se chevauchent suivant la direction axiale de l'adaptateur (10) d'au moins 50% d'une longueur axiale d'au moins l'un des espaces (24, 28) libres,
la paroi (30) de couplage ayant au moins une fenêtre optique de sortie optique d'image, constituée pour transmettre du rayonnement dans le domaine des longueurs d'onde optique.

2. Adaptateur (10) suivant la revendication 1, qui est étanche aux germes entre le côté proximal de l'adaptateur (10) et le côté distale de l'adaptateur(10), pour maintenir séparé ainsi un côté stérile d'un côté non stérile et maintenir ainsi stérile le côté stérile.

3. Adaptateur (10) suivant la revendication 1 ou 2, dans lequel au moins l'un des espaces (24, 28) libres a une section transversale variable suivant la direction axiale et/ou l'angle polaire.

4. Adaptateur (10) suivant au moins l'une des revendications 1 à 3, dans lequel l'espace (24, 28) libre, constitué pour le couplage à au moins une partie d'une extrémité (36) distale de tête de caméra, a au moins une surface (56, 56', 58) de couplage, constitué pour se coupler à au moins une surface (72) de couplage de l'extrémité (36) distale de la tête de caméra et
dans lequel l'espace (24, 28) libre, constitué pour le couplage à au moins une partie d'une extrémité (38) proximale d'endoscope a au moins une surface (56, 56', 58) de couplage, constituée pour se coupler à au moins une surface (68) de couplage de l'extrémité (38) proximale d'endoscope.

5. Adaptateur (10) suivant la revendication 4, dans lequel au moins l'une des surfaces (56, 56', 58) de couplage est de forme cylindrique ou en forme d'enveloppe de cylindre creux.

6. Adaptateur (10) suivant au moins l'une des revendications 4 ou 5, dans lequel au moins l'une des surfaces (56, 56', 58) de couplage est constituée de manière à donner, dans au moins l'un des espaces (24, 28) libres, une section transversale variant suivant la direction axiale.

7. Adaptateur (10) suivant au moins l'une des revendications 1 à 6, qui a un revêtement stérile pour recouvrir la tête (12) de la caméra, le revêtement étant constitué pour entourer de manière stérile la tête (12) de la caméra.

8. Adaptateur (10) suivant au moins l'une de revendications 1 à 7, dans lequel la paroi intérieure a, à la fois sur sa face intérieure et sur sa face extérieure, des surfaces (56, 58) de couplage, constituées pour coupler l'endoscope (14) à la tête (12) de la caméra.

9. Adaptateur (10) suivant au moins l'une des revendications 1 à 8, qui a
au moins une ligne optique de transmission de lumière d'éclairage et/ou de transmission optique de signal et/ou au moins une ligne électrique de transmission de signaux électriques et/ou d'énergie électrique entre le côté proximal de l'adaptateur (10) et le côté distal de l'adaptateur (10).

10. Utilisation d'un adaptateur (10) suivant au moins l'une des revendications 1 à 9, pour le couplage d'un endoscope (14) stéréo à une tête (12) de caméra.
